# EUROPEAN PATENT SPECIFICATION

(11) **EP 2 986 217 B1**
(45) Date of publication and mention of the grant of the patent: **22.11.2017**
(21) Application number: 14722951.2
(22) Date of filing: 14.04.2014
(51) Int. Cl.: A61B 5/15, A61B 5/151, A61B 5/157, A61M 1/34, B01L 3/00, B04B 7/08, G01N 33/49

(54) **BIOLOGICAL FLUID COLLECTION DEVICE AND BIOLOGICAL FLUID SEPARATION SYSTEM**
ENTNAHMEVORRICHTUNG FÜR BIOLOGISCHE FLÜSSIGKEIT SOWIE TRENNUNGSSYSTEM FÜR BIOLOGISCHE FLÜSSIGKEIT
DISPOSITIF DE PRÉLÈVEMENT DE FLUIDE BIOLOGIQUE ET SYSTÈME DE SÉPARATION DE FLUIDE BIOLOGIQUE

(30) Priority: 15.04.2013 US 201361811918 P
(43) Date of publication of application: 24.02.2016
(60) Divisional application: 17193109.0
(73) Proprietor: Becton, Dickinson and Company, Franklin Lakes, NJ 07417-1880 (US)
(72) Inventor: WILKINSON, Bradley, M., North Haledon, New Jersey 07508 (US); GELFAND, Craig, A., Jackson, New Jersey 08527 (US)
(74) Representative: dompatent von Kreisler Selting Werner - Partnerschaft von Patent- und Rechtsanwälten mbB
(86) International application number: PCT/US2014/033923
(87) International publication number: WO 2014/172235

(56) References cited:
- EP-A2- 1 627 651
- WO-A1-93/09710
- WO-A1-2009/123592
- WO-A1-2011/040874
- DE-U1-202008 010 918
- US-A- 5 055 203
- US-A- 5 636 640
- US-A1- 2006 240 964
- US-A1- 2012 277 697

## Description

### BACKGROUND OF THE INVENTION

### 1. Field of the Disclosure

The present disclosure relates generally to devices, assemblies, and systems adapted for use with vascular access devices. More particularly, the present disclosure relates to devices, assemblies, and systems adapted for collecting biological samples.

### 2. Description of the Related Art

Blood sampling is a common health care procedure involving the withdrawal of at least a drop of blood from a patient. Blood samples are commonly taken from hospitalized, homecare, and emergency room patients either by finger stick, heel stick, or venipuncture. Blood samples may also be taken from patients by venous or arterial lines. Once collected, blood samples may be analyzed to obtain medically useful information including chemical composition, hematology, or coagulation, for example.

Blood tests determine the physiological and biochemical states of the patient, such as disease, mineral content, drug effectiveness, and organ function. Blood tests may be performed in a clinical laboratory or at the point-of-care near the patient. One example of point-of-care blood testing is the routine testing of a patient's blood glucose levels which involves the extraction of blood via a finger stick and the mechanical collection of blood into a diagnostic cartridge. Thereafter, the diagnostic cartridge analyzes the blood sample and provides the clinician a reading of the patient's blood glucose level. Other devices are available which analyze blood gas electrolyte levels, lithium levels, and ionized calcium levels. Some other point-of-care devices identify markers for acute coronary syndrome (ACS) and deep vein thrombosis/pulmonary embolism (DVT/PE).

Despite the rapid advancement in point-of-care testing and diagnostics, blood sampling techniques have remained relatively unchanged. Blood samples are frequently drawn using hypodermic needles or vacuum tubes attached to a proximal end of a needle or a catheter assembly. In some instances, clinicians collect blood from a catheter assembly using a needle and syringe that is inserted into the catheter to withdraw blood from a patient through the inserted catheter. These procedures utilize needles and vacuum tubes as intermediate devices from which the collected blood sample is typically withdrawn prior to testing. These processes are thus device intensive, utilizing multiple devices in the process of obtaining, preparing, and testing blood samples. Each additional device increases the time and cost of the testing process.

Point-of-care testing devices allow for a blood sample to be tested without needing to send the blood sample to a lab for analysis. Thus, it is desirable to create a device that provides an easy, safe, reproducible, and accurate process with a point-of-care testing system.

Technical background information is disclosed in the following documents: US 5,636,640, WO 93/09710, DE 20 2008 010 918 and us 2012/0277697.

### SUMMARY OF THE INVENTION

The present disclosure provides a biological fluid separation system, such as a blood separation system, for a blood sample. The biological fluid separation system includes a biological fluid collection device, such as a blood collection device, adapted to receive a blood sample and a centrifuge. The centrifuge is adapted to receive the biological fluid collection device such that with the biological fluid collection device received within the centrifuge and a rotational force applied to the biological fluid collection device, a plasma portion of the blood sample is separated from a cellular portion of the blood sample. The biological fluid collection device is only receivable within the centrifuge in one orientation.

Some of the advantages of the biological fluid collection device and the biological fluid separation system of the present disclosure over prior systems are that it is a closed system which reduces blood sample exposure and it provides passive and fast mixing of the blood sample with a sample stabilizer. Also, a blood sampling transfer device of the present disclosure incorporates the concepts of lancing, blood collection, and blood separation.

In accordance with an embodiment of the present invention, a biological fluid collection device includes a housing having an inlet port and a serpentine flow channel in fluid communication with the inlet port. The device also includes a puncturing element disposed within the housing which is adapted for movement between a pre-actuated position, wherein the puncturing element is retained within the housing, and a puncturing position, wherein the puncturing element extends through the inlet port of the housing and establishes flow communication with the serpentine flow channel.

In certain configurations, when the puncturing element is in the puncturing position, the biological fluid collection device is adapted to generate a vacuum in communication with the inlet port. The device may also include a second flow channel in fluid communication with the serpentine channel, with the second flow channel oriented in a plane that is offset from a plane defining a flow axis of the serpentine channel. The biological fluid collection device may also be adapted to receive a multi-component blood sample having a cellular portion and a plasma portion. When rotational force is applied to the biological fluid collection device, the plasma portion may be separated from the cellular portion through the flow channel.

In certain configurations, the device also includes an indicator element which is transitionable between an initial setting and a complete setting. The indicator element may automatically transition to the complete setting when collection of the blood sample is complete. Optionally, the puncturing element is a micro-needle array. The housing may also include a push button and actuation of the push button may move the puncturing element from the pre-actuated position to the puncturing position. In certain configurations, at least a portion of the serpentine flow channel includes a sample stabilizer. The housing may also include an electric contact for engagement with a corresponding electric contact of a centrifuge.

In accordance with another embodiment of the present invention, a biological fluid separation system for a blood sample having a cellular portion and a plasma portion includes a biological fluid collection device adapted to receive the blood sample. The blood collection device includes a housing having an inlet port and a flow channel in fluid communication with the inlet port, and a puncturing element disposed within the housing. The puncturing element is adapted for movement between a pre-actuated position, wherein the puncturing element is retained within the housing, and a puncturing position, wherein the puncturing element extends through the inlet port of the housing and establishes flow communication with the flow channel. The system also includes an electric contact disposed on an exterior portion of the housing.

In certain configurations, the system also includes a centrifuge having a receiving port adapted to receive the blood collection device. The electric contact of the biological fluid collection device is engaged with a corresponding portion of the centrifuge. The flow channel may be a serpentine flow channel. In some configurations, the biological fluid collection device is only receivable within the centrifuge in one orientation. When the blood collection device is received within the centrifuge and a rotational force applied to the blood collection device, the plasma portion of the blood sample may be separated from the cellular portion through the flow channel.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
**Fig. 1** is a perspective view of a biological fluid collection device in accordance with an embodiment of the present invention.
**Fig. 2** is an elevation view of a biological fluid collection device in accordance with an embodiment of the present invention.
**Fig. 3** is a perspective view of a biological fluid collection device secured to a patient in accordance with an embodiment of the present invention.
**Fig. 4** is a perspective view of a biological fluid separation system in accordance with an embodiment of the present invention.
**Fig. 5** is an elevation view of a blood separation device in accordance with an embodiment of the present invention.
**Fig. 6** is an elevation view of a blood separation device in accordance with an embodiment of the present invention, with a biological fluid collection device received within a receiving port of the blood separation device.
**Fig. 7** is a partial cross-sectional view of a biological fluid collection device in accordance with an embodiment of the present invention.
**Fig. 8** is a cross-sectional view of the biological fluid collection device of Fig. 3 in accordance with an embodiment of the present invention, with a puncturing element in a puncturing position.
**Fig. 9** is a cross-sectional view of the biological fluid collection device of **Fig. 3** with a blood sample received within the biological fluid collection device in accordance with an embodiment of the present invention, with a puncturing element in a pre-actuation position.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

Various point-of-care testing devices are known in the art. Such point-of-care testing devices include test strips, glass slides, diagnostic cartridges, or other testing devices for testing and analysis. Test strips, glass slides, and diagnostic cartridges are point-of-care testing devices that receive a blood sample and test that blood for one or more physiological and biochemical states. There are many point-of-care devices that use cartridge based architecture to analyze very small amounts of blood bedside without the need to send the sample to a lab for analysis. This saves time in getting results over the long run but creates a different set of challenges versus the highly routine lab environment. Examples of such testing cartridges include the i-STAT® testing cartridge from the Abbot group of companies. Testing cartridges such as the i-STAT® cartridges may be used to test for a variety of conditions including the presence of chemicals and electrolytes, hematology, blood gas concentrations, coagulation, or cardiac markers. The results of tests using such cartridges are quickly provided to the clinician.

However, the samples provided to such point-of-care testing cartridges are currently manually collected with an open system and transferred to the point-of-care testing cartridge in a manual manner that often leads to inconsistent results, or failure of the cartridge leading to a repeat of the sample collection and testing process, thereby negating the advantage of the point-of-care testing device. Accordingly, a need exists for a system for collecting, transferring, and testing a sample that provides safer, reproducible, and more accurate results.

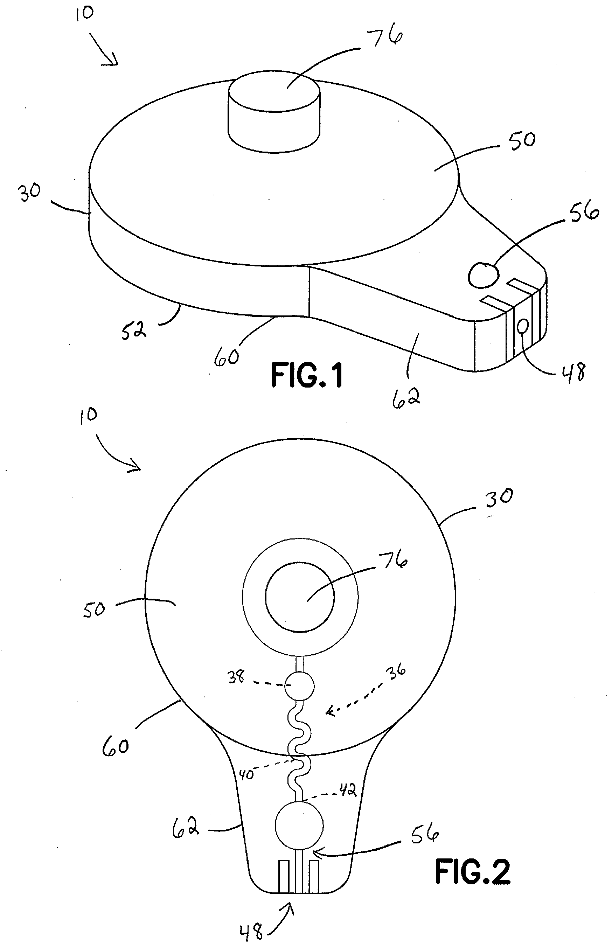

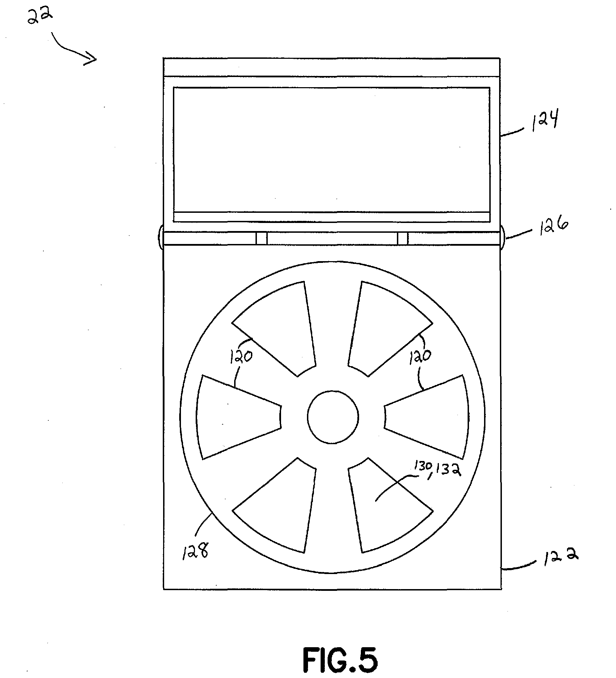

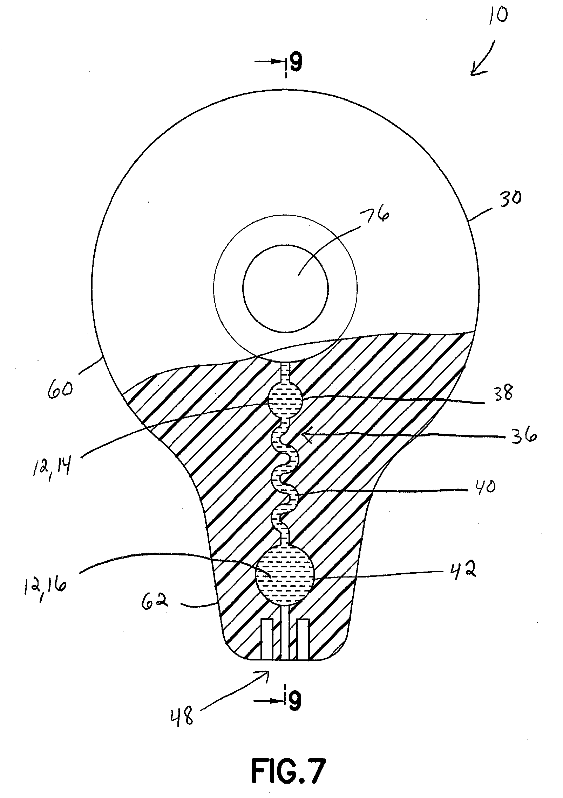

**Figs. 1-9** illustrate an exemplary embodiment of the present disclosure. A biological fluid collection device, such as a blood collection device **10**, of the present disclosure incorporates the concepts of lancing, blood collection, and blood separation. Referring to **Figs. 1-9****,** a blood collection device **10** of the present disclosure is adapted to receive a blood sample **12** having a cellular portion **14** and a plasma portion **16**.

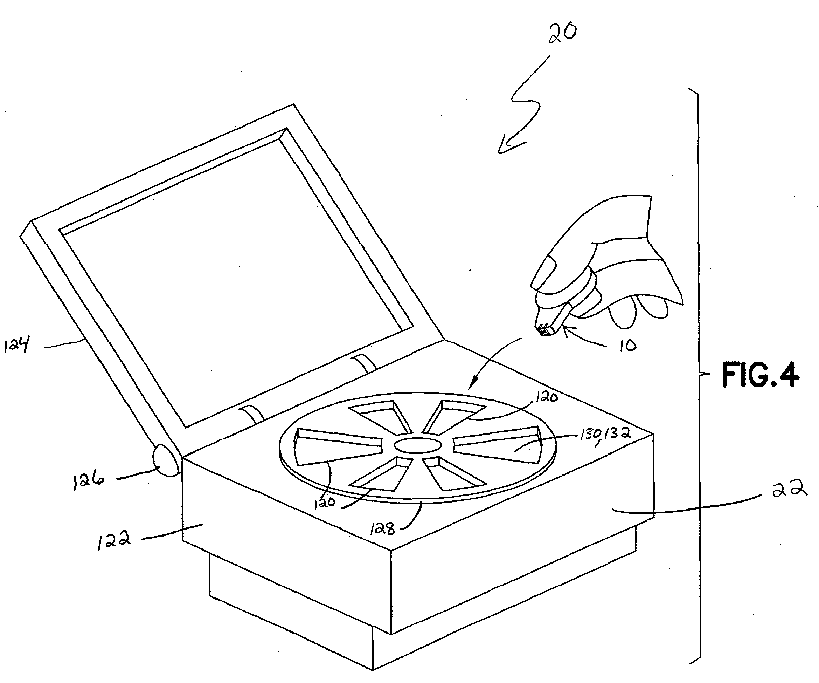

**Figs. 4-6** illustrate an exemplary embodiment of the present disclosure. Referring to **Figs. 4-6**, a biological fluid separation system, such as a blood separation system **20** of the present disclosure for a blood sample **12** includes a blood collection device **10** adapted to receive a blood sample **12** and a blood separation device or centrifuge **22.** The centrifuge **22** is adapted to receive the blood collection device **10** such that with the blood collection device **10** received within the centrifuge **22** and a rotational force applied to the blood collection device **10**, a plasma portion **16** of the blood sample **12** is separated from a cellular portion **14** of the blood sample **12.** The separated blood can then be analyzed by the centrifuge **22** without removing the blood collection device **10** from the centrifuge **22.** For example, a blood separation system of the present disclosure may be used to determine a hematocrit value by centrifugation. The centrifuge **22** may be connected to a computer system and the results of the analysis can be viewed on a display screen of the computer system or sent wirelessly to a hand-held electronic device.

Some of the advantages of the blood collection device and the biological fluid separation system of the present disclosure over prior systems are that it is a closed system which reduces blood sample exposure and it provides passive and fast mixing of the blood sample with a sample stabilizer. Also, a biological fluid sampling transfer device of the present disclosure incorporates the concepts of lancing, blood collection, and blood separation.

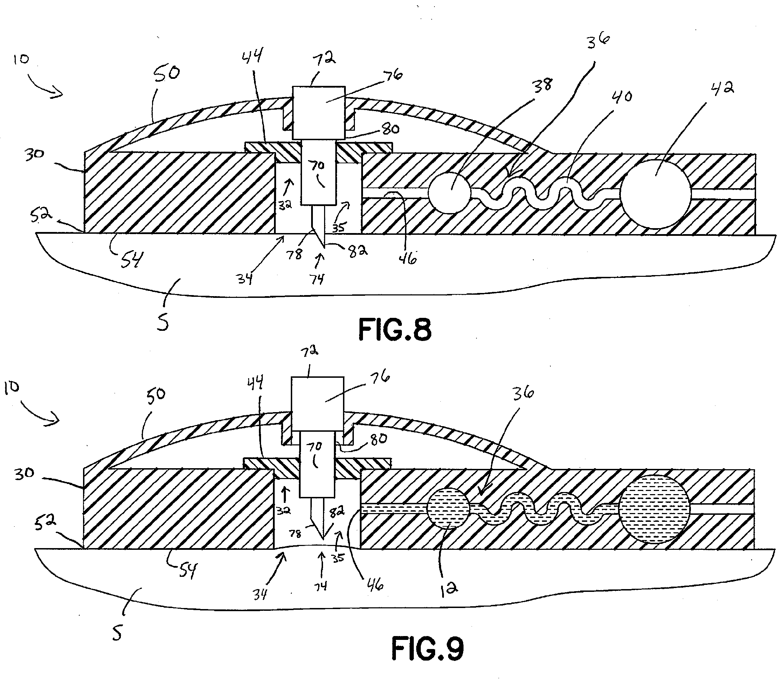

Referring to **Figs. 1-9****,** the blood collection device **10** generally includes a housing 30 defining a central aperture **32** therethrough, an inlet port **34**, an inlet flow channel **35**, a flow **channel 36** in fluid communication with the inlet port **34**, a first reservoir **38** in fluid communication with the inlet port **34,** a serpentine flow channel **40** in fluid communication with the first reservoir **38**, a second reservoir **42** in fluid communication with the serpentine flow channel **40**, a puncturing element engagement portion **44**, a sample stabilizer **46**, an electric contact **48**, and an indicator element **56**. Referring to **Figs. 8** and **9**, the blood collection device **10** includes a puncturing element structure **70** that is positioned within the central aperture **32** of the housing **30**. The housing **30** includes a puncturing element engagement portion **44** for securing the puncturing element structure **70** within the central aperture **32** of the housing **30.** In one embodiment, the inlet flow channel **35** is in fluid communication with the serpentine flow channel **40** and the inlet flow channel **35** is oriented in a plane that is offset from a plane defining a flow axis of the serpentine channel **40.** In one embodiment, the housing **30** of the blood collection device **10** includes the electric contact **48** for engagement with a corresponding electric contact of the centrifuge **22.** In one embodiment, a portion of the housing **30** of the blood collection device **10** is transparent. For example, a portion of the housing **30** is transparent to allow a user to be able to see the cellular portion **14** and the plasma portion **16** within the reservoirs **38**, **42** and the flow channels **36**, **40.**

The blood collection device **10** is adapted to receive a blood sample **12** having a cellular portion **14** and a plasma portion **16**. In one embodiment, the indicator element **56** is transitionable between an initial setting and a complete setting and the indicator element **56** automatically transitions to the complete setting when collection of the blood sample **12** within the blood collection device **10** is complete.

The blood collection device **10** is adapted to contain a sample stabilizer **46** to provide passive and fast mixing of a blood sample with the sample stabilizer **46**. The sample stabilizer **46** can be an anticoagulant, or a substance designed to preserve a specific element within the blood such as, for example, RNA, protein analyte, or other element. In one embodiment, the sample stabilizer **46** is provided within a portion of the flow channel **36.** In other embodiments, the sample stabilizer **46** is provided in other areas of the housing **30** such as the inlet port **34** or the first reservoir **38.**

In one embodiment, a portion of the flow channel **36** comprises a serpentine shape to promote efficient mixing and separation of a blood sample **12** having a cellular portion **14** and a plasma portion **16.** As discussed below, a centrifuge **22** provides a rotational force applied to the blood collection device **10** to separate the plasma portion **16** from the cellular portion **14** through the flow channel **36.** In other embodiments, the flow channel **36** comprises other shapes to promote efficient mixing and separation of a blood sample.

The upper portion of the housing **30** includes a dome-shaped surface **50** and the lower portion of the housing **30** includes a bottom surface **52**. Referring to **Figs. 8** and **9**, the bottom surface **52** includes an adhesive **54** so that the blood collection device **10** can be secured onto a skin surface **S** of a patient where a blood sample will be accessed. In one embodiment, the adhesive **54** of the bottom surface **52** is protected by a peel-off layer, similar to an adhesive bandage, which would be removed before placing the blood collection device **10** on the skin surface S of the patient's body. A hydrogel or other layer could be included to provide some thickness to the bottom surface **52** and help improve the stability of the adhesive seal. Additionally, in one embodiment, the adhesive **54** could include a chemistry to create a more liquid-tight seal, similar to painter's tape technology, where wetting from the paint itself causes a chemical reaction with the adhesive **54** to create a more water-tight barrier to prevent the paint from seeping under the tape.

Referring to **Figs. 1-9****,** the blood collection device **10** also includes a puncturing element structure **70** that may be secured within the central aperture **32** of the housing **30**. The puncturing element structure **70** generally includes a first end **72**, a second end **74**, a push button **76** adjacent the first end **72,** a puncturing element **78** adjacent the second end **74**, and a housing engagement portion **80.** The housing engagement portion **80** engages the puncturing element engagement portion **44** of the housing **30** for securing the puncturing element structure **70** to the housing **30** within the central aperture **32** as shown in **Figs. 8** and **9**. The puncturing element structure **70** includes a puncturing element **78** having a puncturing end **82.** The puncturing end **82** is adapted for puncturing the skin surface **S** of a patient (**Fig. 8**), and may define a pointed end, a blade edge, or a similar cutting mechanism. The puncturing end **82** may include a preferred alignment orientation, such as with a pointed end of a blade aligned in a specific orientation. In one embodiment, the puncturing element **78** comprises a micro-needle array.

The puncturing element **78** is adapted for movement between a pre-actuated position (**Fig. 9**) wherein the puncturing element **78** including the puncturing end **82** is retained within the housing **30** and a puncturing position (**Fig. 8**) wherein the puncturing end **82** of the puncturing element **78** extends through the inlet port **34** of the housing **30** to puncture a skin surface **S** of a patient to draw a blood sample and to establish flow communication with the flow channel **36.** In one embodiment, actuation of the push button **76** moves the puncturing element **78** from the pre-actuated position (**Fig. 9**) to the puncturing position (**Fig. 8**).

In one embodiment, with the puncturing element **78** in the puncturing position, the blood collection device **10** is adapted to generate a vacuum in communication with the inlet port **34** of the housing **30** of the blood collection device **10** to assist in pulling the blood sample **12** within the blood collection device **10**.

In one embodiment, the housing **30** of the blood collection device **10** may include a self-sealing dock that would allow an external lancet or puncturing element to be removably received within the housing **30**. The external lancet or puncturing element could be either preintegrated into the packaged blood collection device **10** or introduced separately by a user before using the blood collection device **10** of the present disclosure.

Referring to **Figs. 4-6**, a blood separation system **20** of the present disclosure for a blood sample **12** includes a blood collection device **10** adapted to receive a blood sample **12** and a blood separation device or centrifuge **22**. The centrifuge **22** is adapted to receive the blood collection device **10** such that with the blood collection device **10** received within the centrifuge **22** and a rotational force applied to the blood collection device **10**, a plasma portion **16** of the blood sample **12** is separated from a cellular portion **14** of the blood sample **12**. The separated blood can then be analyzed by the centrifuge **22** without removing the blood collection device **10** from the centrifuge **22.** The centrifuge **22** may be connected to a computer system and the results of the analysis can be viewed on a display screen of the computer system or sent wirelessly to a hand-held electronic device.

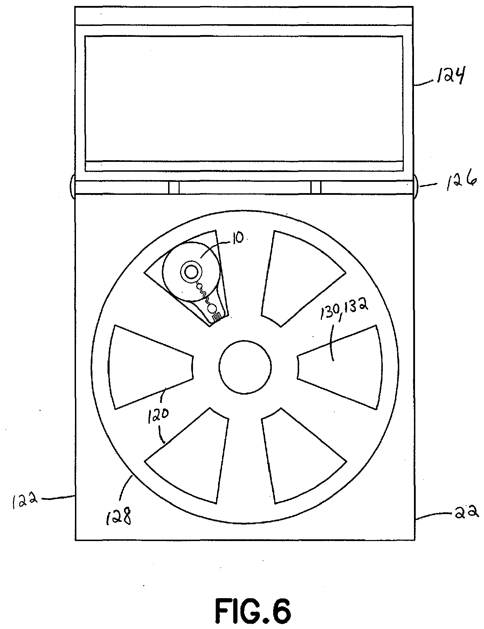

Referring to **Figs. 4-6**, a blood separation device or centrifuge **22** of the present disclosure generally includes a receiving port **120** adapted to receive the blood collection device **10** such that with the blood collection device **10** received within the centrifuge **22** and a rotational force applied to the blood collection device **10**, a plasma portion **16** of the blood sample **12** is separated from a cellular portion **14** of the blood sample **12**. The centrifuge **22** includes a receiving port **120** adapted to receive the blood collection device **10**, a base or bottom portion **122**, a top portion **124** movably connected to the base portion **122** by a hinged portion **126**, and a rotational force element **128** contained within the base portion **122**. The top portion **124** is transitionable between an open position in which the blood collection device **10** can be placed within the receiving port **120** as shown in **Fig. 6** and a closed position. With the blood collection device **10** received within the centrifuge **22**, a rotational force is applied to the blood collection device **10** to separate the plasma portion **16** from the cellular portion **14**.

Referring to **Fig. 4**, in one embodiment, the centrifuge **22** includes a plurality of receiving ports **120** each adapted to receive a separate blood collection device **10**. In this manner, the blood separation system **20** of the present disclosure can receive, separate, and analyze a plurality of blood collection devices **10**.

Referring to **Figs. 1-6****,** a blood collection device **10** of the present disclosure is only receivable within a receiving port **120** of the centrifuge **22** in one orientation. In one embodiment, the housing **30** of the blood collection device **10** has an exterior profile **60** including a key portion **62.** The receiving port **120** of the centrifuge **22** defines an interior profile **130** including a keyway portion **132.** The exterior profile **60** and the key portion **62** of the blood collection device **10** are sized and shaped to substantially correspond to the interior profile **130** and the keyway portion **132** of the receiving port **120** of the centrifuge **22** such that the blood collection device **10** is only receivable within the receiving port **120** of the centrifuge **22** in one orientation. In this manner, the blood collection device **10** can only be inserted into the receiving port **120** of the centrifuge **22** in one way to ensure that the electric contact **48** provided on the blood collection device **10** is placed properly and is engaged with a corresponding portion of the centrifuge **22.**

The exterior profile **60** and the key portion **62** of the blood collection device **10** and the interior profile **130** and the keyway portion **132** of the receiving port **120** of the centrifuge 22 can have any shape that provides a key portion and a keyway portion that only allow the blood collection device **10** to be received within the receiving port **120** of the centrifuge **22** in one orientation.

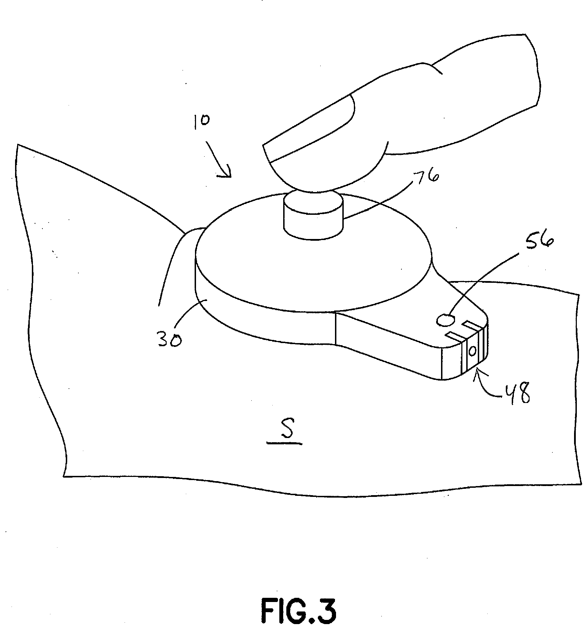

Referring to **Figs. 1-9**, use of a blood collection device of the present disclosure will now be described. Referring to **Figs. 3** and **9**, upon selecting a site, a clinician can adhere the adhesive **54** on the bottom surface **52** of the housing **30** onto a skin surface **S** of a patient where a blood sample will be accessed over a selected sampling site.

Next, the push button **76** on the blood collection device **10** is depressed or actuated to move the puncturing element **78** from the pre-actuated position (**Fig. 9**) to the puncturing position (**Fig. 8**) so that the puncturing element **78** punctures the skin surface **S of** a patient. In one embodiment, a vacuum is applied simultaneously. Thereafter, a blood sample **12** is drawn into a microfluidic flow channel **36** via capillary action. The blood sample is exposed to and mixed with a sample stabilizer **46** in the flow channel **36**. The sample stabilizer **46** can be an anticoagulant, or a substance designed to preserve a specific element within the blood such as, for example, RNA, protein analyte, or other element.

When the indicator element **56** turns to a complete setting or a specific color such as red, indicating that collection of the blood sample **12** has been completed, the user removes the blood collection device **10** from the patient's arm.

Referring to **Figs. 4-6**, the next step of the process involves manual insertion of the blood collection device **10** into a blood separation device or centrifuge **22** designed specifically for the blood collection device **10.** For example, the blood collection device **10** is transferred to a "spin chip" blood separation device or centrifuge **22** that acts as a miniaturized smart blood separation device. The blood collection device **10** can only be inserted into the receiving port **120** of the centrifuge **22** in one way to ensure that the electric contact **48** provided on the blood collection device **10** is placed properly and is engaged with a corresponding portion of the centrifuge **22**.

The centrifuge **22** is designed to facilitate plasma separation by centrifugal force and to drive a blood sample through the flow channel **36** of the blood collection device **10.** The blood sample **12** contained within the blood collection device **10** is quickly spun in the centrifuge **22** and due to the low volume is separated through the flow channel **36** of the blood collection device **10** within a few seconds such that the plasma portion **16** is collected within the second reservoir **42** of the blood collection device **10**.

In one embodiment, the centrifuge **22** is adapted to receive the blood collection device **10** such that with the blood collection device **10** received within the centrifuge **22** and a rotational force applied to the blood collection device **10**, a plasma portion **16** of the blood sample **12** is separated from a cellular portion **14** of the blood sample **12.** The separated blood can then be analyzed by the centrifuge **22** without removing the blood collection device **10** from the centrifuge **22.** The centrifuge **22** may be connected to a computer system and the results of the analysis can be viewed on a display screen of the computer system or sent wirelessly to a hand-held electronic device.

Some of the advantages of the blood collection device and the blood separation system of the present disclosure over prior systems are that it is a closed system which reduces blood sample exposure and it provides passive and fast mixing of the blood sample with a sample stabilizer. Also, the blood sampling transfer device of the present disclosure incorporates the concepts of lancing, blood collection, and blood separation.

## Claims

1. A biological fluid collection device, **characterized by**:
a housing (30) having an inlet port (34) and a serpentine flow channel (40) in fluid communication with the inlet port (34); and
a puncturing element (78) disposed within the housing (30) and adapted for movement between a pre-actuated position wherein the puncturing element (78) is retained within the housing (30) and a puncturing position wherein the puncturing element (78) extends through the inlet port (34) of the housing and establishes flow communication with the serpentine flow channel (40).

2. The biological fluid collection device of claim 1, wherein with the puncturing element (78) in the puncturing position, the biological fluid collection device is adapted to generate a vacuum in communication with the inlet port (34).

3. The biological fluid collection device of claim 1, further comprising a second flow channel in fluid communication with the serpentine flow channel (40), the second flow channel oriented in a plane that is offset from a plane defining a flow axis of the serpentine flow channel (40).

4. The biological fluid collection device of claim 1, wherein the biological fluid collection device (10) is adapted to receive a multi-component blood sample (12) having a cellular portion (14) and a plasma portion (16).

5. The biological fluid collection device of claim 4, wherein when a rotational force is applied to the biological fluid collection device (10), the plasma portion (16) is separated from the cellular portion (14) through the serpentine flow channel (40).

6. The biological fluid collection device of claim 4, further comprising an indicator element (56) transitionable between an initial setting and a complete setting, wherein the indicator element (56) automatically transitions to the complete setting when collection of the blood sample (12) is complete.

7. The biological fluid collection device of claim 1, wherein the puncturing element (78) comprises a micro-needle array.

8. The biological fluid collection device of claim 1, wherein the housing (30) includes a push button (76), wherein actuation of the push button (76) moves the puncturing element (78) from the pre-actuated position to the puncturing position.

9. The biological fluid collection device of claim 1, wherein at least a portion of the serpentine flow channel (40) comprises a sample stabilizer.

10. The biological fluid collection device of claim 1, wherein the housing (30) includes an electric contact (48) for engagement with a corresponding electric contact of a centrifuge (22).

11. A biological fluid separation system (20) for a blood (12) sample having a cellular portion (14) and a plasma portion (16), comprising:
a biological fluid collection device (10) adapted to receive the blood sample (12), the biological fluid collection device (10) **characterized by**:
a housing (30) having an inlet port (34) and a flow channel (40) in fluid communication with the inlet port (34);
a puncturing element (78) disposed within the housing (30) and adapted for movement between a pre-actuated position wherein the puncturing element is retained within the housing and a puncturing position wherein the puncturing element (78) extends through the inlet port (34) of the housing (30) and establishes flow communication with the flow channel (40); and
an electric contact (48) disposed on an exterior portion of the housing (30) for engaging a portion for a centrifuge (22)

12. The biological fluid separation system of claim 11, further comprising a centrifuge (22) having a receiving port (120) adapted to receive the biological fluid collection device (10), wherein the electric contact (48) of the biological fluid collection device (10) is engaged with the portion of the centrifuge (22).

13. The biological fluid separation system of claim 11, wherein the flow channel is a serpentine flow channel (40).

14. The biological fluid separation system of claim 11, wherein the biological fluid collection device (10) is only receivable within the centrifuge (22) in one orientation.

15. The biological fluid separation system of claim 11, wherein with the biological fluid collection device (10) received within the centrifuge (22) and a rotational force applied to the biological fluid collection device (10), the plasma portion (16) of the blood sample (12) is separated from the cellular portion (14) through the flow channel (40).

## Patentansprüche

1. Sammelvorrichtung für biologisches Fluid, **gekennzeichnet durch**:
ein Gehäuse (30) mit einem Einlassport (34) und einem serpentinenförmigen Durchflusskanal (40), der in Fluidverbindung mit dem Einlassport (34) steht; und
ein Punktionselement (78), das in dem Gehäuse (30) angeordnet ist und geeignet ist, zwischen einer vorbetätigten Position, in welcher das Punktionselement (78) im Inneren des Gehäuses (30) gehalten ist, und einer Punktionsposition bewegbar ist, in welcher das Punktionselement (78) sich durch den Einlassport (34) des Gehäuses erstreckt und die Fluidverbindung mit dem serpentinenförmigen Durchflusskanal (40) herstellt.

2. Sammelvorrichtung für biologisches Fluid nach Anspruch 1, bei welcher, bei in der Punktionsposition befindlichem Punktionselement (78), die Sammelvorrichtung für biologisches Fluid geeignet ist, ein in Verbindung mit dem Einlassport (34) stehendes Vakuum zu erzeugen.

3. Sammelvorrichtung für biologisches Fluid nach Anspruch 1, ferner mit einem zweiten Durchflusskanal in Fluidverbindung mit dem serpentinenförmigen Durchflusskanal (40), wobei der zweite Durchflusskanal in einer Ebene ausgerichtet ist, die gegenüber einer Ebene versetzt ist, welche eine Durchflussachse des serpentinenförmigen Durchflusskanals (40) ist.

4. Sammelvorrichtung für biologisches Fluid nach Anspruch 1, bei welcher die Sammelvorrichtung (10) für biologisches Fluid geeignet ist, eine Multi-Komponenten-Blutprobe (12) aufzunehmen, die einen Zellanteil (14) und einen Plasmaanteil (16) aufweist.

5. Sammelvorrichtung für biologisches Fluid nach Anspruch 4, bei welcher, wenn eine Drehkraft auf die Sammelvorrichtung (10) für biologisches Fluid aufgebracht wird, der Plasmaanteil (16) von dem Zellanteil (14) durch den serpentinenförmigen Durchflusskanal (40) getrennt wird.

6. Sammelvorrichtung für biologisches Fluid nach Anspruch 4, ferner mit einem Indikatorelement (56), das zwischen einer Anfangseinstellung und einer Abschlusseinstellung bewegbar ist, wobei das Indikatorelement (56) automatisch zur Abschlusseinstellung übergeht, wenn das Sammeln der Blutprobe (12) abgeschlossen ist.

7. Sammelvorrichtung für biologisches Fluid nach Anspruch 1, bei welcher das Punktionselement (78) eine Mikronadelanordnung aufweist.

8. Sammelvorrichtung für biologisches Fluid nach Anspruch 1, bei welcher das Gehäuse (30) einen Druckknopf (76) aufweist, wobei die Betätigung des Druckknopfs (76) das Punktionselement (78) von der vorbetätigten Position in die Punktionsposition bewegt.

9. Sammelvorrichtung für biologisches Fluid nach Anspruch 1, bei welcher mindestens ein Bereich des serpentinenförmigen Durchflusskanals (40) eine Probenstabilisator aufweist.

10. Sammelvorrichtung für biologisches Fluid nach Anspruch 1, bei welcher das Gehäuse (30) einen elektrischen Kontakt (48) zum Zusammengreifen mit einem entsprechenden elektrischen Kontakt einer Zentrifuge (22) aufweist.

11. System (20) zum Trennen von biologischem Fluid für eine Blutprobe (12), die einen Zellanteil (14) und einen Plasmaanteil (16) aufweist, mit:
eine Sammelvorrichtung (10) für biologisches Fluid, die zur Aufnahme der Blutprobe (12) geeignet ist, wobei die Sammelvorrichtung (10) für biologisches Fluid **gekennzeichnet ist durch**:
ein Gehäuse (30) mit einem Einlassport (34) und einem Durchflusskanal (40), der in Fluidverbindung mit dem Einlassport (34) steht;
ein Punktionselement (78), das in dem Gehäuse (30) angeordnet ist und geeignet ist, zwischen einer vorbetätigten Position, in welcher das Punktionselement (78) im Inneren des Gehäuses (30) gehalten ist, und einer Punktionsposition bewegbar ist, in welcher das Punktionselement (78) sich durch den Einlassport (34) des Gehäuses (30) erstreckt und die Fluidverbindung mit dem Durchflusskanal (40) herstellt; und
einen elektrischen Kontakt (48), der an einem äußeren Bereich des Gehäuses (30) angeordnet ist, um mit einem Bereich einer Zentrifuge (22) zusammenzugreifen.

12. System zum Trennen von biologischem Fluid nach Anspruch 11, ferner mit einer Zentrifuge (22), die einen Aufnahmeport (120) aufweist, der zum Aufnehmen der Sammelvorrichtung (10) für biologisches Fluid geeignet ist, wobei der elektrische Kontakt (48) der Sammelvorrichtung (10) für biologisches Fluid mit dem Bereich der Zentrifuge (22) zusammengreift.

13. System zum Trennen von biologischem Fluid nach Anspruch 11, bei welchem der Durchflusskanal ein serpentinenförmiger Durchflusskanal (40) ist.

14. System zum Trennen von biologischem Fluid nach Anspruch 11, bei welchem die Sammelvorrichtung (10) für biologisches Fluid nur in einer Ausrichtung in der Zentrifuge (22) aufnehmbar ist.

15. System zum Trennen von biologischem Fluid nach Anspruch 11, bei welchem, wenn die Sammelvorrichtung (10) für biologisches Fluid in der Zentrifuge (22) aufgenommen ist und eine Drehkraft auf die Sammelvorrichtung (10) für biologisches Fluid aufgebracht wird, der Plasmaanteil (16) der Blutprobe (12) von dem Zellanteil (14) durch den Durchflusskanal (40) getrennt wird.

## Revendications

1. Dispositif de prélèvement de fluide biologique, **caractérisé par** :
un boîtier (30) ayant un orifice d'entrée (34) et un canal d'écoulement en serpentin (40) en communication fluidique avec l'orifice d'entrée (34) ; et
un élément de ponction (78) disposé dans le boîtier (30) et adapté pour se déplacer entre une position pré-actionnée dans laquelle l'élément de ponction (78) est retenu dans le boîtier (30) et une position de ponction dans laquelle l'élément de ponction (78) s'étend à travers l'orifice d'entrée (34) du boîtier et établit une communication d'écoulement avec le canal d'écoulement en serpentin (40).

2. Dispositif de prélèvement de fluide biologique de la revendication 1, dans lequel, avec l'élément de ponction (78) dans la position de ponction, le dispositif de prélèvement de fluide biologique est adapté pour générer un vide en communication avec l'orifice d'entrée (34).

3. Dispositif de prélèvement de fluide biologique de la revendication 1, comprenant en outre un deuxième canal d'écoulement en communication fluidique avec le canal d'écoulement en serpentin (40), le deuxième canal d'écoulement étant orienté dans un plan qui est décalé par rapport à un plan définissant un axe d'écoulement du canal d'écoulement en serpentin (40).

4. Dispositif de prélèvement de fluide biologique de la revendication 1, dans lequel le dispositif de prélèvement de fluide biologique (10) est adapté pour recevoir un échantillon de sang à composants multiples (12) ayant une partie cellulaire (14) et une partie plasmatique (16).

5. Dispositif de prélèvement de fluide biologique de la revendication 4, dans lequel, lorsqu'une force de rotation est appliquée au dispositif de prélèvement de fluide biologique (10), la partie plasmatique (16) est séparée de la partie cellulaire (14) par le canal d'écoulement en serpentin (40).

6. Dispositif de prélèvement de fluide biologique de la revendication 4, comprenant en outre un élément indicateur (56) pouvant effectuer une transition entre un réglage initial et un réglage complet, où l'élément indicateur (56) passe automatiquement au réglage complet lorsque le prélèvement de l'échantillon de sang (12) est achevé.

7. Dispositif de prélèvement de fluide biologique de la revendication 1, dans lequel l'élément de ponction (78) comprend un réseau de micro-aiguilles.

8. Dispositif de prélèvement de fluide biologique de la revendication 1, dans lequel le boîtier (30) comporte un bouton-poussoir (76), où l'actionnement du bouton-poussoir (76) permet de déplacer l'élément de ponction (78) de la position pré-actionnée à la position de ponction.

9. Dispositif de prélèvement de fluide biologique de la revendication 1, dans lequel au moins une partie du canal d'écoulement en serpentin (40) comprend un stabilisateur d'échantillon.

10. Dispositif de prélèvement de fluide biologique de la revendication 1, dans lequel le boîtier (30) comporte un contact électrique (48) pour s'engager avec un contact électrique correspondant d'une centrifugeuse (22).

11. Système de séparation de fluide biologique (20) pour un échantillon de sang (12) ayant une partie cellulaire (14) et une partie plasmatique (16), comprenant :
un dispositif de prélèvement de fluide biologique (10) adapté pour recevoir l'échantillon de sang (12), le dispositif de prélèvement de fluide biologique (10) étant **caractérisé par** :
un boîtier (30) ayant un orifice d'entrée (34) et un canal d'écoulement (40) en communication fluidique avec l'orifice d'entrée (34) ;
un élément de ponction (78) disposé dans le boîtier (30) et adapté pour se déplacer entre une position pré-actionnée dans laquelle l'élément de ponction est retenu dans le boîtier et une position de ponction dans laquelle l'élément de ponction (78) s'étend à travers l'orifice d'entrée (34) du boîtier (30) et établit une communication d'écoulement avec le canal d'écoulement (40) ; et
un contact électrique (48) disposé sur une partie externe du boîtier (30) pour s'engager avec une partie pour une centrifugeuse (22).

12. Système de séparation de fluide biologique de la revendication 11, comprenant en outre une centrifugeuse (22) ayant un orifice de réception (120) adapté pour recevoir le dispositif de prélèvement de fluide biologique (10), où le contact électrique (48) du dispositif de prélèvement de fluide biologique (10) est engagé avec la partie de la centrifugeuse (22).

13. Système de séparation de fluide biologique de la revendication 11, dans lequel le canal d'écoulement est un canal d'écoulement en serpentin (40).

14. Système de séparation de fluide biologique de la revendication 11, dans lequel le dispositif de prélèvement de fluide biologique (10) ne peut être reçu dans la centrifugeuse (22) que dans une orientation.

15. Système de séparation de fluide biologique de la revendication 11, dans lequel, avec le dispositif de prélèvement de fluide biologique (10) reçu dans la centrifugeuse (22) et une force de rotation appliquée au dispositif de prélèvement de fluide biologique (10), la partie plasmatique (16) de l'échantillon de sang (12) est séparée de la partie cellulaire (14) par le canal d'écoulement (40).
